# EUROPEAN PATENT SPECIFICATION

(11) **EP 2 063 816 B1**
(45) Date of publication and mention of the grant of the patent: **12.08.2015**
(21) Application number: 07833020.6
(22) Date of filing: 21.09.2007
(51) Int. Cl.: A61L 27/04, A61F 2/28, C22C 23/00, A61L 27/30, A61L 27/58, A61F 2/06, A61F 2/30, A61F 2/36, A61F 2/44, A61B 17/68, A61B 17/80

(54) **IMPLANTS COMPRISING BIODEGRADABLE METALS AND METHOD FOR MANUFACTURING THE SAME**
IMPLANTATE MIT BIOLOGISCH ABBAUBAREN METALLEN UND HERSTELLUNGSVERFAHREN DAFÜR
IMPLANTS COMPRENANT DES MÉTAUX BIODÉGRADABLES ET PROCÉDÉ DE PRODUCTION DE CES DERNIERS

(30) Priority: 22.09.2006 KR 20060092308; 22.09.2006 KR 20060092309
(43) Date of publication of application: 03.06.2009
(73) Proprietor: U & I Corporation, Uijeongbu-si, Gyeonggi-do 480-050 (KR)
(72) Inventor: YANG, Seok-Jo, Daejeon Metropolitan City 300-717 (KR); SEOK, Hyun-Kwang, Seoul 135-283 (KR); KIM, Jung-Gu, Gyeonggi-do, 463-010 (KR); LIM, Tae-Hong, Seoul 137-072 (KR); BAIK, Kyeong-Ho, Daejeon Metropolitan City, 305-358 (KR); KIM, Yu-Chan, Seoul, 142-779 (KR); KOO, Ja-Kyo, Seoul, 139-220 (KR)
(74) Representative: Eisenführ Speiser
(86) International application number: PCT/KR2007/004650
(87) International publication number: WO 2008/035948

(56) References cited:
- WO-A2-2007/125532
- US-A1- 2005 266 041
- US-A1- 2006 039 947
- US-A1- 2006 064 160
- US-A1- 2006 085 081
- US-B2- 6 495 156
- DATABASE WPI Week 200643 Thomson Scientific, London, GB; AN 2006-416150 XP002640483, & CN 1 743 486 A (TANG Z) 8 March 2006 (2006-03-08)
- OKAMOTO H.: "Ca-Mg (Calcium-Magnesium)", JOURNAL OF PHASE EQUILIBRIA, vol. 19, no. 5, 1 October 1998 (1998-10-01), pages 490-490, DOI: 10.1361/105497198770342012

## Description

### [Technical Field]

The present invention relates to implants and a method for manufacturing the same. More specifically, the present invent ion relates to implants comprising biodegradable materials, in which their biodegradation rate can be easily controlled, and they have excellent strength and interfacial strength to an osseous tissue, thereby being used as a bone substitute or for bone treatment, and a method for manufacturing the same.

### [Background Art]

A representative implant material used for medical applications is a metal material having excellent mechanical properties and processability. In spite of the excellent properties of metal, the metallic implants have several problems such as stress shielding, image degradation, and implant migration.

In order to overcome these problems of the metallic implants, the development of biodegradable implants has been suggested. Polymers including polylactic acids (PLA), polyglycolic acids (PGA), and PLGA that is copolymers thereof have been studied from the middle of 1960s, as the biodegradable materials in medical applications. However, the above mentioned biodegradable polymers have problems such as lower mechanical strength, acid generation on degradation, and difficulty in controlling their biodegradation rate, whereby their applications have been limited. In particular, there is a limitation in the application of the polymers to orthopedic or dental implant requiring load-bearing capacities due to the property of lower mechanical strength.

In order to overcome such problems of the biodegradable polymers, studies have been conducted on several biodegradable materials, for example, ceramic such as tri-calcium phosphate (TCP) and composite material of biodegradable polymer and biodegradable hydroxyapatite (HA). However, the mechanical properties of the materials have not been greatly improved as compared to the biodegradable polymers, and the weak impact resistance of ceramic material has been thought to be a serious drawback as a biomaterial. Further, the control of biodegradable materials or the like has not been clearly explained yet, thus their practical applications are still problematic.

On the other hand, in order to overcome problems of metallic implants, studies on surface modification of metallic implants by a coating method has been tried, in addition to studies on the implant material itself. There are two main objects in the surface modification of metallic implants by coating technology. First, it is an object to provide improvements in wear resistance or corrosion resistance of interface between metallic implant and metal or non-metal material, for example, DLC (Diamond-Like Carbon) coating or the like. Second, it is an object to strengthen interfacial adhesion strength between metallic implant and osseous tissue, which can be achieved by coating the metallic implant with a material having high adhesion strength to osseous tissue. In this connection, a material generally used is hydroxyapatite (HA), which is similar to a bone component. Further, in order to improve adhesion strength to osseous tissue, the implant can be coated by using bone cement (PMMA).

Among them, HA has excellent biocompatibility, as well as similar components and structure to osseous tissue. Thus, it has been known to have excellent interfacial adhesion strength to osseous tissue by chemical bonding. While HA has excellent chemical bonding strength to osseous tissue, HA has lower interfacial adhesion strength to implant. Therefore, HA particles detached from the implant surface have been considered as a serious problem. In total hip-replacement, the detached HA was found in polyethylene acetabular cup, and severe frictional wear of the acetabular cup by HA and osseointegration by worn polyethylene were observed.

Accordingly, many methods have been tried in order to improve the adhesion strength between HA and implant, one of which is the improvement of coating method. A general method used in HA coating is a plasma spraying technique. In this method, crystalline HA is converted to amorphous calcium phosphate phase and deposited during spraying of HA particles, and the coating materials mechanically adhere to the implant. Therefore, adhesion strength is low and HA calcium phosphate particles are easily detached. In order to solve these problems, a variety of methods have been tried, but practical problems still remain. Further, there are a lot of technical problems such as maintenance of crystalline phase, coating thickness and uniformity. From the viewpoint of materials, the detachment of HA particles are mainly caused by chemical incompatibility between HA coating material, which is one of ceramics, and metallic implant material. Accordingly, there are still certain limitations in their interfacial adhesion strength, in spite of efforts to improve the coating technology.
In connection with the present invention, reference is also made to CN 1743486A which discloses an alloy with Mg as the matrix and the use as a fixer for fractures.

### [Disclosure]

### [Technical Problem]

The present invention provides implants having biodegradability, in which their biodegradation rate can be easily controlled, and they has excellent strength and interfacial strength to an osseous tissue, and a method for manufacturing the same, in order to solve the above-described problems of the prior art, which are the problems of the known metallic implant and biodegradable polymer implant.

### [Technical Solution]

In order to achieve the objects, the present invention provides implants according to the attached claims 1 to 7, comprising biodegradable magnesium-based alloys. The implant according to one embodiment of the present invention consists of a magnesium-based alloy, see claim 3. The implant according to another embodiment of the present invention is constituted with a coating layer consisting of biodegradable magnesium-based alloys provided on the implant surface, see claim 4.

The biodegradable magnesium-based alloy is represented by Formula MgₐCa_{b}X_{c} as defined in claim 1.

Provided that nickel (Ni) is added, the content of nickel (Ni) is preferably 100 ppm or less, and more preferably 50 ppm or less, in order to reduce biotoxicity and control the corrosion rate. In the case of adding iron (Fe), iron (Fe) greatly affects the corrosion rate of magnesium-based alloy. Further, even if a trace amount of iron (Fe) is contained with magnesium (Mg), iron (Fe) is not employed in magnesium (Mg) and is present as separate particles to increase the corrosion rate of magnesium (Mg). While magnesium (Mg) is decomposed in a living body, the individual iron (Fe) particles present in the magnesium-based alloy can flow into the living body. Therefore, the content of iron (Fe) should be precisely determined, preferably 1,000 ppm or less, and more preferably 500 ppm or less.

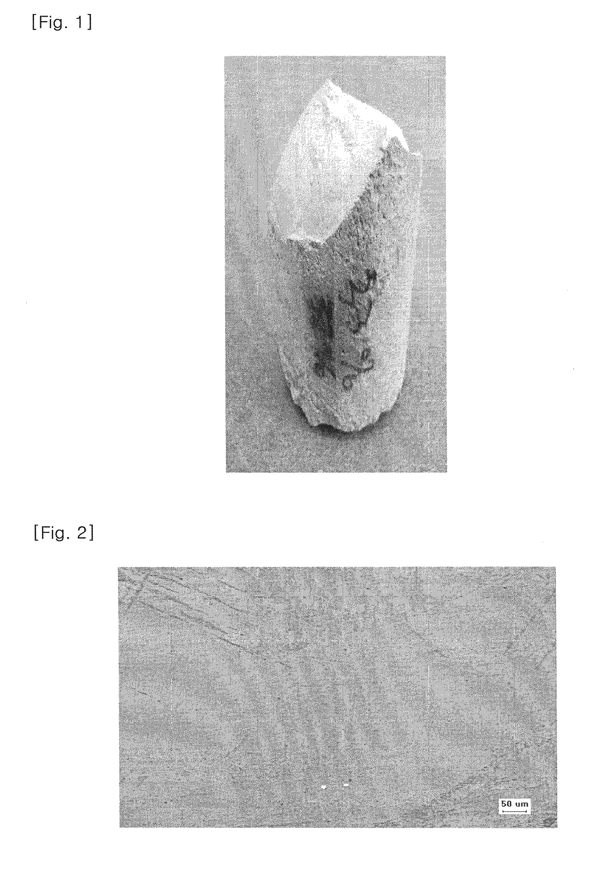

Further, in the case of adding a large amount of second and third elements including calcium (Ca) to magnesium (Mg), a precipitated phase or intermediate compound having high brittleness is formed in the alloy. Therefore, the alloy material may fracture in the process for manufacturing the implant, and easily fracture in the second process such as extrusion and forging. Further, the material easily fractures in lathe processing for manufacturing the implant product, resulting in difficult processing. Fig. 1 is a photograph showing the appearance of Mg-33%Ca alloy material that is cast by adding 33% calcium (Ca) to pure magnesium (Mg) containing 0.001% iron (Fe) and 0.0035% nickel (Ni) as impurities. It can be seen that after casting, the upper end of the alloy material fractured. Subsequently, the alloy material was broken into pieces in the handling and cutting process. Further, in order to perform its extrusion process, the extrusion temperature should be increased to 450°C or more. Accordingly, in the case where the addition of the second and third elements is 40% or more, its practical application is not thought to be effective. In the present invention, the addition of the second and third elements is limited to 0.4 (40%) or less.

The present invention provides a method for manufacturing implants using biodegradable magnesium-based alloys. The method for manufacturing implants according to one embodiment of the present invention comprises the steps of: melting biodegradable magnesium-based alloys, and molding the molten biodegradable magnesium-based alloys. The method for manufacturing implants according to another embodiment of the present invention comprises the step of coating the implant surface with the magnesium-based alloys.

### [Advantageous Effects]

In the implants comprising the biodegradable magnesium-based alloys according to the present invention, high strength magnesium-based alloys have twice or more higher strength than a known biodegradable polymer, but the degrees are different depending on the composition and manufacturing process of alloys. Thus, high strength magnesium-based alloys are applied to materials for osseointegration in the lumbar region requiring high load-bearing capacity and dental implants, and they can be suitably used to maintain initial stability. Further, the implant according to the present invention degrades in a body and osseous tissue simultaneously grows into the implant. Therefore, excellent interfacial strength is provided between implant and osseous tissue, and the biodegradation rate can be easily controlled to proceed in proportion to the degree of forming osseous tissue, whereby the stability is not lost before osseointegration and ion release that is suddenly generated due to degradat ion in the body can be control led. As a result, bone format ion can stably occur.

On the other hand, the implant having a coating layer consisting of biodegradable magnesium-based alloys according to the present invention has excellent properties with respect to strength, interfacial strength between implant and osseous tissue, and control of its biodegradation rate by the coating layer. Additionally, in the case of using a metal material as the base material of the implant, the implant has excellent interfacial adhesion strength between coating layer and implant, since both the coating layer consisting of magnesium-based alloys and the base material of metallic implant are metal.

Accordingly, the implant according to the present invention can be suitably used as a bone substitute or for bone treatment, and used as orthopedic, dental, plastic surgical, or vascular implants.

### [Description of Drawings]

Fig. 1 is a photograph showing the appearance of Mg_{0.67}Ca_{0.33} casting alloy;
Fig. 2 is a photograph showing a cross-section of pure Mg (Fe 0.001 to 0.04%, Ni 0.0035 to 0.001%) casting alloy;
Fig. 3 is a photograph showing a cross-sect ion of Mg_{0.992}Ca_{0.008} alloy;
Fig. 4 is a photograph showing a cross-section of Mg_{0.95}Ca_{0.05} alloy;
Fig. 5 is a photograph showing a cross-sect ion of Mg_{0.895}Ca_{0.105} alloy;
Fig. 6 is a photograph showing a cross-section of Mg_{0.77}Ca_{0.23} alloy;
Fig. 7 is a photograph showing a cross-sect ion of Mg_{0.67}Ca_{0.33} alloy;
Fig. 8 is a photograph showing a cross-section of a sample, which is a rapid quenched Mg_{0.67}Ca_{0.33} alloy by a gas blowing method;
Fig. 9 is a photograph showing a longitudinal and horizontal cross-section of Mg_{0.95}Ca_{0.05} casting alloy extruded;
Fig. 10 is a result of compressive strength test on Mg casting alloys prepared by adding different amounts of Ca;
Fig. 11 is a result of compressive strength test on extruded Mg casting alloys prepared by adding different amounts of Ca;
Fig. 12 is a result of measuring the changes in corrosion current density of Mg material according to impurity content, addition amount of Ca, and extrusion processing;
Fig. 13 illustrates the changes in corrosion current density and yield strength of Mg material according to impurity content, addition amount of Ca, and extrusion processing;
Fig. 14 is a schematic diagram showing surface modification of metallic implant using a biodegradable magnesium-based alloy and effects thereof;
Fig. 15 is a cross-sectional view of a coating layer consisting of magnesium-based alloy that is formed on the surface of Ti alloy implant by a sputtering;
Fig. 16 is a view of the scratched coating layer consisting of magnesium-based alloy that is formed on the surface of Ti alloy implant by a sputtering;
Fig. 17 is a photograph showing bone formation in the region, where the magnesium-based alloy is degraded, when the magnesium-based alloy manufactured in Example 2 is applied to in vivo test using a mouse; and
Figs. 18 to 25 are graphs showing the changes in the levels of aspartate liver enzyme (AST), alanine liver enzyme (ALT), creatinine, blood urea nitrogen (BUN), hemoglobin, white blood cell, hematocrit (Hct), and alkaline phosphatase in blood taken from the mouse, to which the magnesium-based alloy manufactured in Example 2 was applied in vivo test.

### [Best Mode]

Hereinafter, the present invention will be described in detail.

The implants according to the present invention have the above-described effects to be used as orthopedic, dental, plastic surgical, or vascular implants. In particular, the implants can be used as an implant for spinal interbody spacer, bone filler, bone plate, bone pin, scaffold, etc.

Magnesium-based alloys and a method for manufacturing an implant using the same will be described in detail as follows.

Magnesium generally ignites at very low temperature (about 450°C) to need specific treatment on melting. In the manufacturing process of commercial magnesium-based alloy, a small amount of Be (10 ppm or less) is added to the magnesium-based alloy, and the surface of molten alloy is coated by using a mixed gas of SF₆, CO₂, and dry air. As such, a compact mixed coating film consisting of MgNₓ, BeO, MgO, MgF₂, MgS or the like is formed on the surface of molten al loy to prevent the molten magnesium-based alloy from reacting with oxygen. Thus, the operation can be stably performed. However, in the case of considering the addition of impurity such as biomaterial, oxide forming elements such as Be cannot be added to the magnesium-based alloy. Therefore, it is preferable that the magnesium-based alloy is molten under an inert gas environment such as argon (Ar) that does not react with the magnesium-based alloy or under vacuum. In order to melt the magnesium-based alloy, a variety of methods including a resistance heating method to generate heat by applying electricity to a resistance, an induction heating method by applying current to an induct ion coat ing, and a method using laser or focusing light can be used, and the resistance heating method is the most economical. Upon melting the magnesium-based alloy, the molten alloy is preferably stirred to mix the elements.

According to one embodiment of the present invention, the magnesium-based alloy molten by the above described method is molded to a shape of implant, thereby providing an implant. A method known in the related art can be used as a method for molding the implant by using the molten magnesium-based alloy. For example, the molten alloy can be solidified by quenching.

In the quenching process, the molten magnesium-based alloy can be rapidly quenched for the purpose of improving the mechanical strength of magnesium-based alloy. At this time, a method for immersing crucible in water can be used. Further, in the quenching process, a method for spraying the magnesium-based alloy using an inert gas such as argon gas can be used, in which the alloy can be quenched at much higher rate to exhibit very fine structure. However, when the magnesium-based alloy is cast in such a small size, a lot of pores (dark parts) can be formed in the alloy.

Further, the molten alloy can be molded using an extrusion process. In this case, the structure of magnesium-based alloy becomes uniform, and the mechanical performance can be improved. The extrusion of the magnesium-based alloy is preferably performed in the temperature range of 300 to 450°C. Further, the extrusion of the magnesium-based alloy can be performed while maintaining the reduction ratio of cross-section (extrusion ratio) before and after extrusion from 10:1 to 30:1. As the extrusion ratio increases, the microstructure of the extruded material becomes uniform, and the defects formed on casting are easily removed. In this case, it is preferable that the capacity of extruder is increased.

In the molding step, a method for molding in the shape of implant can be performed by using a metal processing method known in the related art. For example, the implant with a desired shape and use can be manufactured by a direct casting method, in which the above described molten magnesium-based alloy is poured in a processed mold being closer to the shape of final product, a lathe or milling processing method after manufacturing an intermediate product having a rod or sheet shape, a method for forging the magnesium-based alloy with high pressure to manufacture in the shape of final product or the like.

Further, if necessary, the surface grinding or coating is performed to improve the quality of the manufactured magnesium-based alloy product.

According to another embodiment of the present invention, the implant known in the related art is coated with the magnesium-based alloy molten by the above described method to provide the implant with a coating layer consisting of biodegradable magnesium-based alloys provided on the implant surface.

A variety of methods known in the related art can be used as the method for coating the implant surface with the magnesium-based alloy. For example, an immersion coating method, in which the implant base material is immersed in a crucible containing the molten magnesium-based alloy to coat its surface with the magnesium-based alloy; a solid phase/liquid phase cladding method, in which the implant base material is put in a mold having slightly larger diameter than the implant base material, and then the magnesium-based alloy is injected to the space therebetween to coat the magnesium-based alloy; a continuous solid state/liquid state cladding method of modification of solid state/liquid state cladding method, inwhich the implant base material is passed through the mold having larger diameter than the implant base material, and then the magnesium-based alloy is injected to the space therebetween to continuously coat the magnesium-based alloy; TIG or MIG welding method, in which a magnesium-based alloy wire is manufactured, and then while approaching the implant base material with the magnesium-based alloy wire, current is supplied to melt the magnesium-based alloy wire, and the base material surface is coated; a laser, focusing light or ion beam welding method, in which the magnesium-based alloy powder is put on the surface of implant base material, and heat source such as laser, light and ion beam is applied to melt the magnesium-based alloy powder, and the surface of implant base material is coated; a sputtering method, in which RF (Radio frequency) current, direct current, or ion beam is applied to the magnesium-based alloy material, and the elements of the magnesium-based alloy are emitted in a unit of atom to be deposited on the surface of implant base material can be used as the coat ing method. In the present invention, other coating methods not mentioned in the above list can be used. The suitable coating method can be selected depending on a thickness of the desired coating layer, cleanliness of the coating material, cost or the like. For example, a thick coating layer having a thickness of 100 *µ*m or more can be economical ly coated by using the immersion method. On the other hand, the sputtering method is useful to form a thin film having a thickness of 1 *µ*m or less, and to form a clean coating layer.

Since magnesium ignites at very low temperature (about 450°C), it is preferable that the above-list coating method is performed under vacuum, or by shielding the part to be coated with an inert gas such as argon gas in order to prevent the magnesium-based alloy from contact ing with oxygen.

In the present invention, the implant materials coated with the magnesium-based alloy may be a metal, a biodegradable polymer or a biomaterial, but are not limited thereto. In the case of using a metal as the implant material, a bond between the magnesium-based alloy and metallic implant is a metal-metal bond, of which chemical bond strength is more excellent than that of a HA (ceramic)-metal bond, and the interfacial adhesion strength between magnesium-based alloy and osseous tissue is improved due to the new formation of osseous tissue. In the present invention, the implant surface is preferably washed before coating with the magnesium alloy.

### [Mode for Invention]

Hereinafter, a manufacture of magnesium-based alloys and manufacture of implants using the same will be illustrated with reference to Examples. However, these Examples are for the illustrative purpose only, and the invention is not intended to be limited by these Examples.

### Manufacture of magnesium-based alloy

### Example 1

### Manufacture of implant material using pure magnesium (not according to the invention)

In the case of high pure material with a low content of impurities, as its purity is higher, the manufacturing cost is exponentially increased, whereby its commercial value deteriorates. In Examples, in order to determine an impurity concentration of magnesium available as an implant material, magnesium is manufactured by adding different amounts of Fe and Ni, and the corrosion characteristics are evaluated (hereinafter, Mg with the impurity concentration of 0.01% or less is referred to as pure Mg or 100% Mg). A stainless steel (SUS 410) crucible having an internal diameter of 50 mm was charged with each magnesium, in which ultrapure reagent grade magnesium (99.9999%) is mixed with Fe and Ni of 1) 400 ppm (0.04%), 10 ppm (0.001%), 2) 70 ppm (0.007%), 5 ppm (0.0005%), 3) 10 ppm (0.001%), 35 ppm (0.0035%). Subsequently, in order to prevent magnesium in the crucible from contacting with air, whi le argon (Ar) gas was al lowed to flow around the crucible, a temperature of the crucible was increased to the range of about 700 to 750°C using a resistance heater to melt magnesium. The crucible was stirred to mix well the molten magnesium and impurities. The magnesium completely molten was quenched to prepare solid magnesium. Further, upon quenching, the crucible was immersed in water to rapidly quench the molten magnesium, for the purpose of improving the mechanical strength of magnesium. Fig. 2 is a photograph showing the cross section of ground pure Mg (Fe 0.001 to 0.04%, Ni 0.0035 to 0.001%) casting alloy, which was observed by an optical microscope.

### Example 2

### Manufacture of Mg-Ca alloy

A magnesium-based alloy was manufactured by mixing magnesium with calcium. 0.8%, 5%, 10.5%, 23%, and 33% Ca were mixed with pure Mg (purity 99.995%) having impurities of 10 ppm (0.001%) Fe and 35 ppm (0.0035%) Ni, and a stainless steel (SUS 410) crucible having an internal diameter of 50 mm was charged with the mixed materials. Subsequently, in order to prevent the magnesium-based alloy in the crucible from contacting with air, while argon (Ar) gas was allowed to flow around the crucible, a temperature of the crucible was increased to the range of about 700 to 1000°C using a resistance heater to melt the magnesium-based alloy. The magnesium-based alloy completely molten was quenched to prepare solid magnesium-based alloy. The crucible was stirred to mix the elements of the molten magnesium-based alloy with each other. Further, upon quenching, the crucible was immersed in water to rapidly quench the molten magnesium-based alloy, for the purpose of improving the mechanical strength of magnesium-based alloy.

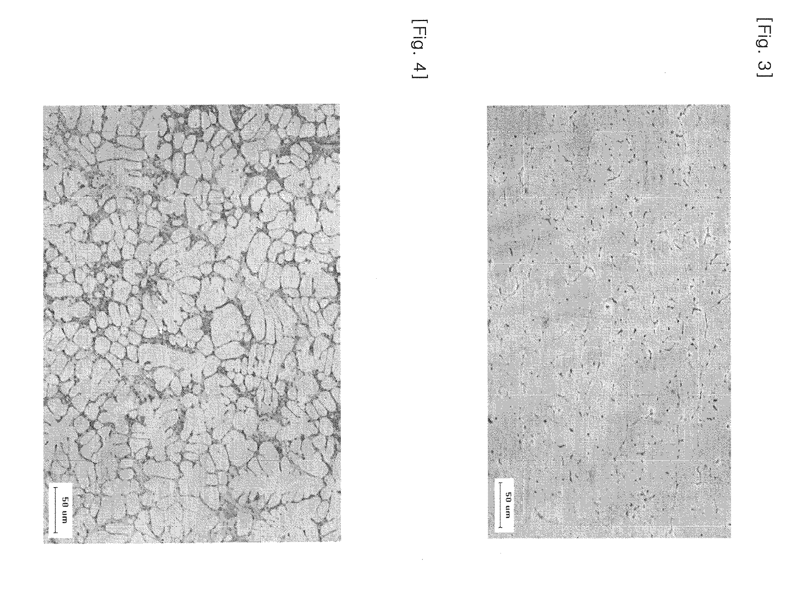

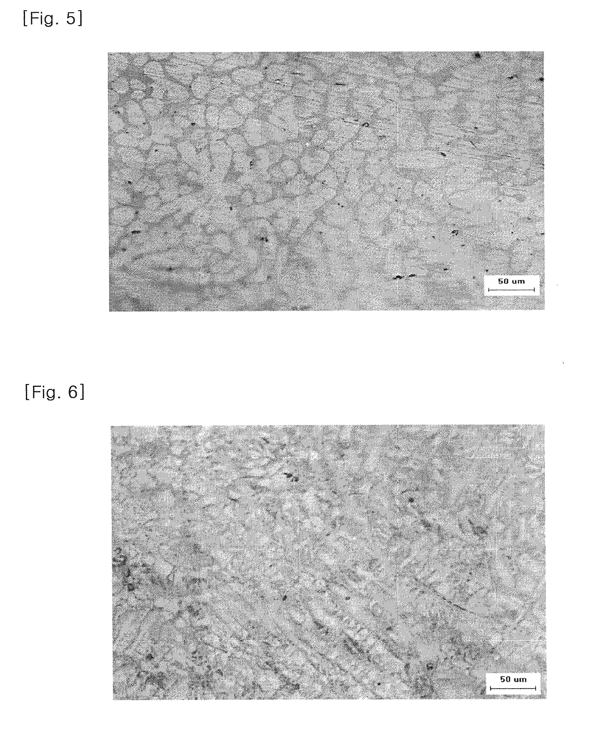

Figs. 3 to 7 are each photograph showing the cross sections of ground Mg_{0.992}Ca_{0.008}, Mg_{0.95}Ca_{0.05}. Mg_{0.895}Ca_{0.105}, Mg_{0.77}Ca_{0.23}, and Mg_{0.67}Ca_{0.33} alloys manufactured by the above mentioned method, which were observed by an optical microscope. In the Mg_{0.992}Ca_{0.008} alloy, Mg with grey color has most of the area, and the mixed area with dark grey color of Mg₂Ca compound and Mg is partially observed. As the amount of Ca increased, the area with dark grey color (referred as the mixed area or processed area of Mg₂Ca compound and Mg) increased. Further, defects such as internal pore were not observed in the all of the manufactured Mg-Ca alloy samples. Therefore, it can be seen that the alloys were well manufactured.

### Example 3

### Manufacture of Mg-Ca alloy by rapid quenching using gas blowing

The magnesium-based alloy was molten with a heater, and then the molten magnesium-based alloy was injected to a fine hole having a diameter of about 3 mm by a spraying method with argon gas, and solidified to manufacture the rapid quenched magnesium-based alloy material. In the case of using this method, the magnesium-based alloy material can be quenched at much higher rate than that of Examples 1 and 2, thereby exhibiting a very fine structure.

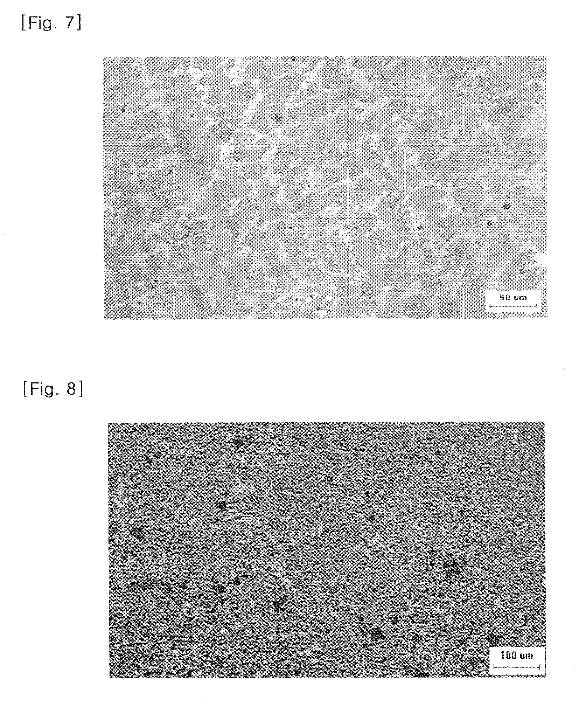

Fig. 8 is a photograph showing the cross sect ion of Mg_{0.67}Ca_{0.33} alloy manufactured by the above described method, which was observed by an opt ical microscope. As compared to Fig. 7, which is an optical photograph showing the cross section of magnesium-based alloy material manufactured by immersing the crucible in water to be quenched, the size of the composition phase is found to be very fine.

### Example 4

### Manufacture of magnesium-based alloy by extrusion

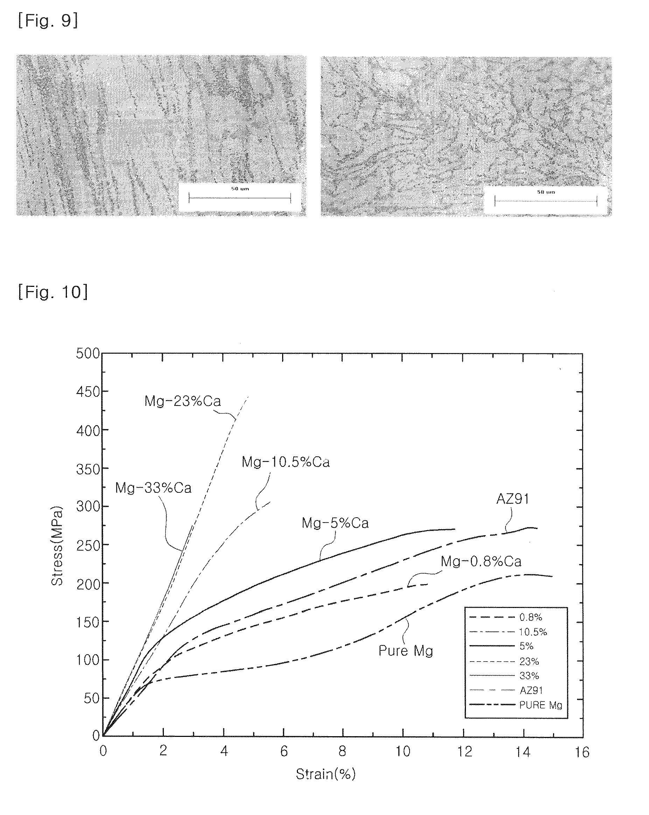

Magnesium-based alloys, in which each has impurities of 0.001% Fe and 0.0035% Ni, and 0%, 0.8%, 5%, 10.5%, 23%, and 33% Ca, respectively, were manufactured by the above described method in Example 1, and then extruded. An extrusion temperature was varied depending on the content of Ca. As the content of Ca was increased, the temperature was increased to easily perform extrusion. The extrusion was performed in the temperature range of 300 to 450°C, the reduction ratio of cross-section (extrusion ratio) before and after extrusion was fixed at 15:1. The changes in microstructure according to extrusion are shown in Fig. 9. Fig. 9 is a photograph showing a longitudinal (left) and horizontal (right) cross-sect ion of the extruded material, which is the Mg-5%Ca cast ing al loy of Fig. 4 extruded by the above method, and observed by an optical microscope. It was found that the rose petal-shaped microstructure shown in the casting alloy of Fig. 4 was deformed on extrusion.

### Test for strength measurement of magnesium-based alloy

In order to test the strength of the magnesium-based al loy material of the present invention, the magnesium-based alloy materials manufactured in Examples 1 and 2 were subjected to electro discharge machining, and processed in a form having a diameter of 3 mm and a length of 6 mm. The lower portion and top portion of the processed samples were polished with a No. 1000 emery paper to adjust the level of the surface. The processed test samples were horizontally placed on a jig made of tungsten carbide, and then a force was vertically applied to the samples using a head of a compression tester with maximum loads of 20 ton. At this time, the vertical speed of head was 10⁻⁴/s. During the test, the changes in strain and compressive stress were recorded in real time using an extensometer and a load cell equipped in the compression tester. At this time, the size of the sample was too small to equip the extensometer in the sample, and the extensometer was equipped in the jig of tester compressing the sample. Therefore, the strain of the sample was measured as higher than its actual strain.

Fig. 10 is a graph showing a result of strength test on magnesium-based alloys of the present invention prepared by adding different amounts of calcium. From the result of Fig. 10, it was found that in the magnesium-based al loy, as the content of calcium was increased, the strength of alloy was increased. On the other hand, it was found that when the content of calcium was increased from 22% to 33%, the magnesium-based alloy was broken at lower stress. It can be seen that the alloy having higher content of calcium is preferably used as the implant material applied to the region of a body requiring a high load-bearing capacity.

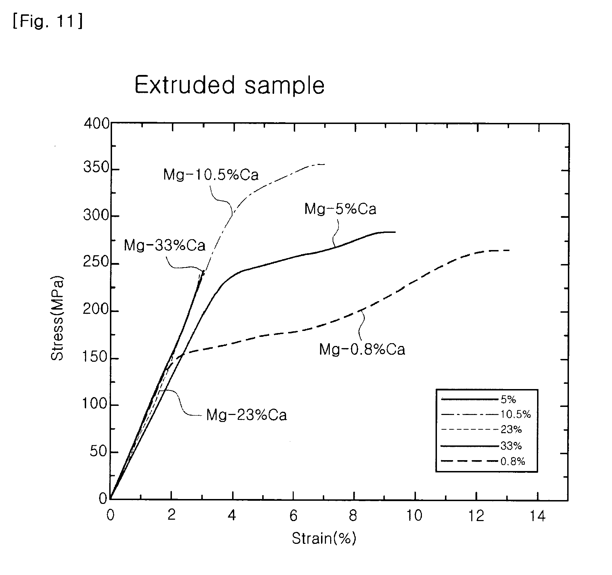

Fig. 11 is a result of measuring the compressive strength of the extruded casting alloy. The yield strengths (stress applied to the material at which material is deformed from straight to curve) of most Mg-Ca alloys were increased by extrusion. However, in the case where the content of calcium was high by 23%, the yield strength was greatly decreased. That is because Mg₂Ca with high brittleness widely distributed in the Mg base structure is broken or separated from the Mg base structure in the extrusion process to exist as defects. In the case where the content of calcium was increased to be 33%, most of alloy materials came to be consisted of Mg₂Ca phase having higher brittleness. Thus, the changes in the strength before and after extrusion were very small.

### Corrosion rate test on magnesium-based alloy

In order to evaluate corrosion characteristics of magnesium-based alloys, a Potentio Dynamic Test was used. First, the magnesium-based alloy material manufactured by casting was cut and ground with a No. 1000 emery paper. The surface area of the ground magnesium-based alloy material except 1 cm² area was coated with an insulating material. Subsequently, the magnesium-based alloy was connected at anode, and Pt and Ag-AgCl as a reference were connected at cathode, and then the anode and cathode were immersed in an etchant to measure the current while gradually increasing voltage. The etchant was consisted of components being similar to human body fluid, and used by mixing the components illustrated in the following Table 1 in 1 liter of water. The temperature of the solution was maintained at 37°C during the test.

**[Table 1]**

| Etchant composition used in corrosion test (based on total weight of 1 liter) | |
|---|---|
| Component | Weight (g) |
| NaCl (Sodium Chloride) | 8 |
| KCl (Potassium Chloride) | 0.4 |
| NaHCO₃(Sodium Hydrogen Carbonate) | 0.35 |
| NaH₂PO₄ · H₂O (A430846 420) | 0.25 |
| Na₂HPO₄ · 2H₂O (K32618380 408) | 0.06 |
| MgCl₂ (Magnesium Chloride) | 0.19 |
| MgSO₄ · 7H₂O (Magnesium Sulfate Heptahydrate) | 0.06 |
| Glucose | 1 |
| CaCl₂ · 2H₂O (Calcium Chloride Dihydrate) | 0.19 |

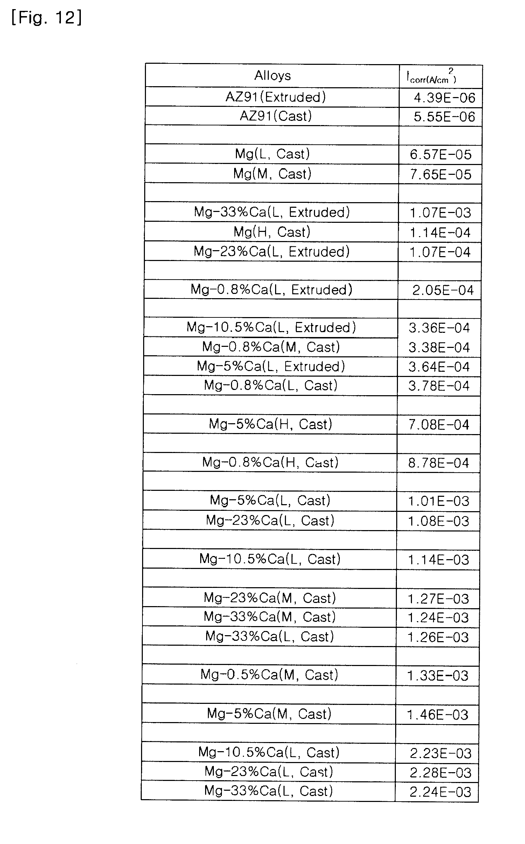

Fig. 12 is a graph showing a result of the corrosion test for magnesium-calcium based alloys prepared by various compositions. In Fig. 12, "cast" means a sample that is cast, "Extruded"?means a sample that is extruded, "H" means a material having impurities of 0.04% Fe and 0.001% Ni, "M" means a material having impurities of 0.07% Fe and 0.0005% Ni, and "L" means a material having impurities of 0.001% Fe and 0.0035% Ni. It was found that in the magnesium-based alloy, as the content of calcium was increased, its corrosion rate was increased. It was found that in pure Mg, as the content of Fe, which is an important factor to determine the corrosion rate, was increased, its corrosion rate was increased. Further, it was found that the microstructure became fine and uniform by extrusion, thereby greatly reducing the corrosion rate. Therefore, it can be seen that the desired corrosion rate can be obtained by controlling processes according to extrusion or the like, impurity concentration, and the amount of Ca as an additional element. As a reference, the corrosion rates of AZ91 (Mg-9%Al-1%Zn) casting alloy and extruded material that are developed and commercially available as a corrosion resistant high strength material are provided as a comparative data. It was found that the corrosion rate close to that of AZ91, which is a material having very excellent corrosion resistance among known Mg alloys, can be obtained by controlling impurities, additional elements, and second process (extrusion or the like).

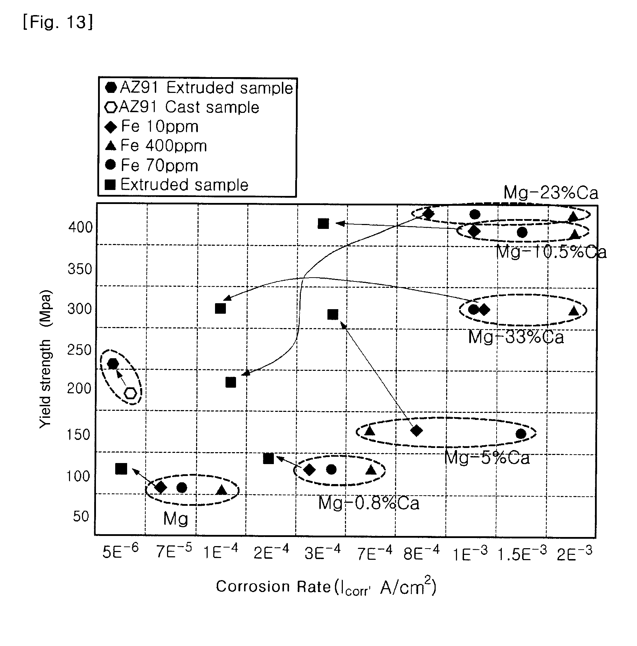

Fig. 13 is a drawing simultaneously illustrating the corrosion rate and strength of Mg al loy. From Fig. 13, the corrosion rate and compressive strength can be controlled by impurity concentration, the addition amount of Ca, and second process such as extrusion in Mg, and a material can be selected according to the type of implant to be applied and its desired characteristics. For example, as the implant product, to which external stress is not great ly appl ied, such as bone filler and bone plate, a variety of materials from low strength material to high strength Mg material can be used. In the case of requiring high strength such as spinal interbody spacer, the high strength Mg containing a predetermined amount of Ca is preferably used.

### Example 5

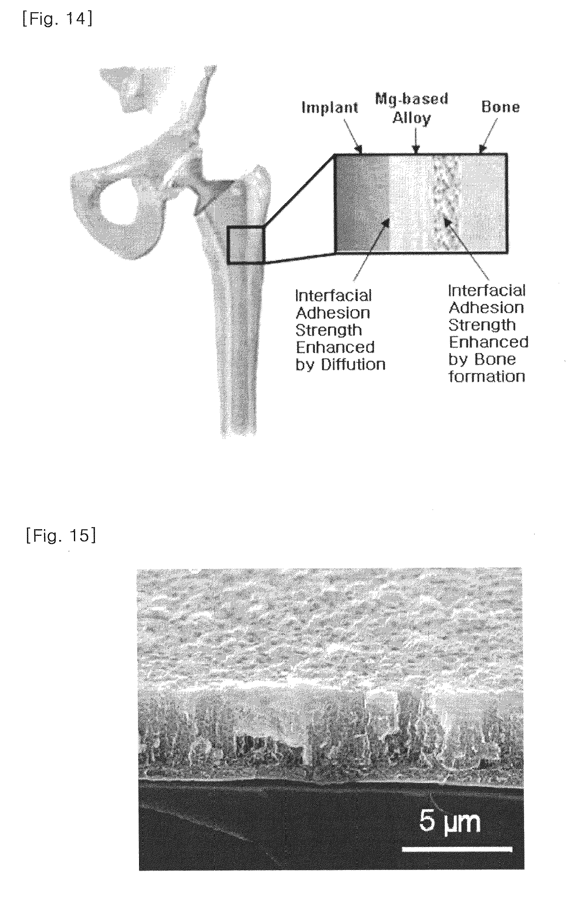

The alloys manufactured in Examples 1 and 2 were processed to be discs (target) having a diameter of 3 inches and a thickness of 5 mm, and then placed in a vacuum chamber. A RF power source was supplied, and a coating layer of magnesium-based alloy was formed on the surface of the base material of Ti alloy by sputtering. As shown in Fig. 14, a implant can be provided with the desired function of bone formation in the present invention by coating the surface of the known implant with the magnesium-based alloy. The implant is as biocompatible as a known biocompatible coating material such as Hydroxyapatite (HA) while preventing from tissue necrosis, which is generated when it has low adhesion strength with a metallic implant and it is separated from the implant after inserting into living body. Fig. 15 is a cross-sectional view of biomaterial (Ti alloy), on which a coating layer is formed by the method, cut by bending impact and observed by an electron microscope. It was found that a thickness of the coating layer is about 5 *µ*m, and the coating layer perpendicularly grows. Further, unevenness was observed on the coating surface of magnesium-based alloy.

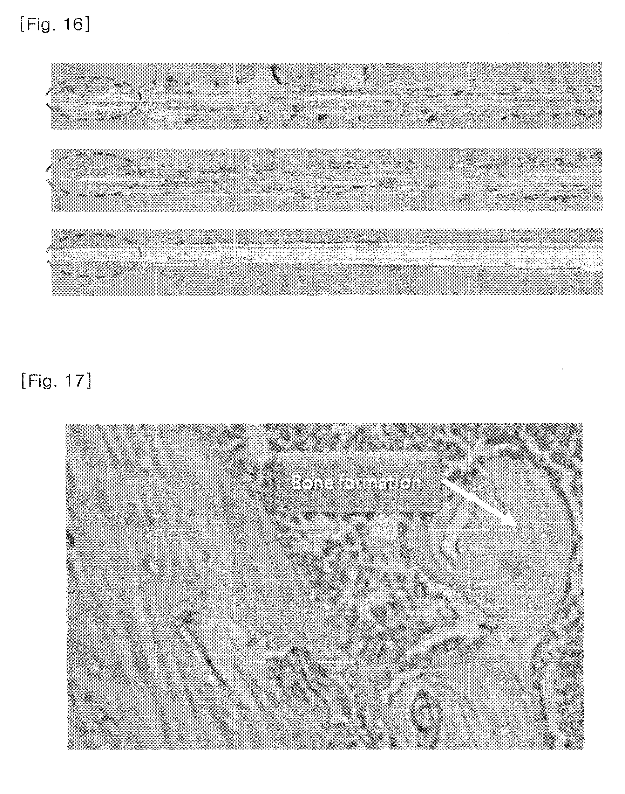

Fig. 16 is a photograph of the surface of the coating layer of magnesium based alloy manufactured by Examples, observed by the electron microscope, in which the coating layer was formed on Ti₆V₄Al alloy used as a biomaterial, and scratched with a diamond tip. In Fig. 16, a top photograph illustrates a sample that was coated without surface washing, a middle photograph illustrates a sample that was coated after surface washing under plasma environment for about 1 minute, and a bottom photograph illustrates a sample that was coated after surface washing under plasma environment for about 2 minute. In the case of coating without surface washing, a little peeling off was found to occur around the surface of coating layer scratched with a diamond tip by external stress. However, in the case of coating after clearly washing the surface, the peeling off was not found to occur on the surface of coating layer by external stress. Accordingly, it can be expected that the total peeling off of the magnesium-based alloy to coat a metallic bio-implant does not occur due to external stress in human body.

### In vivo test

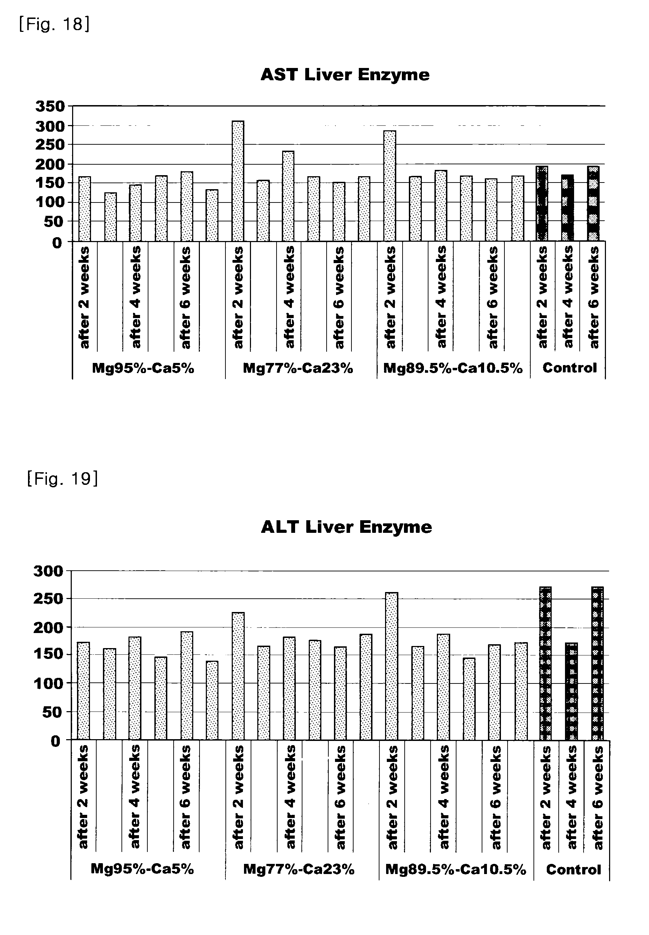

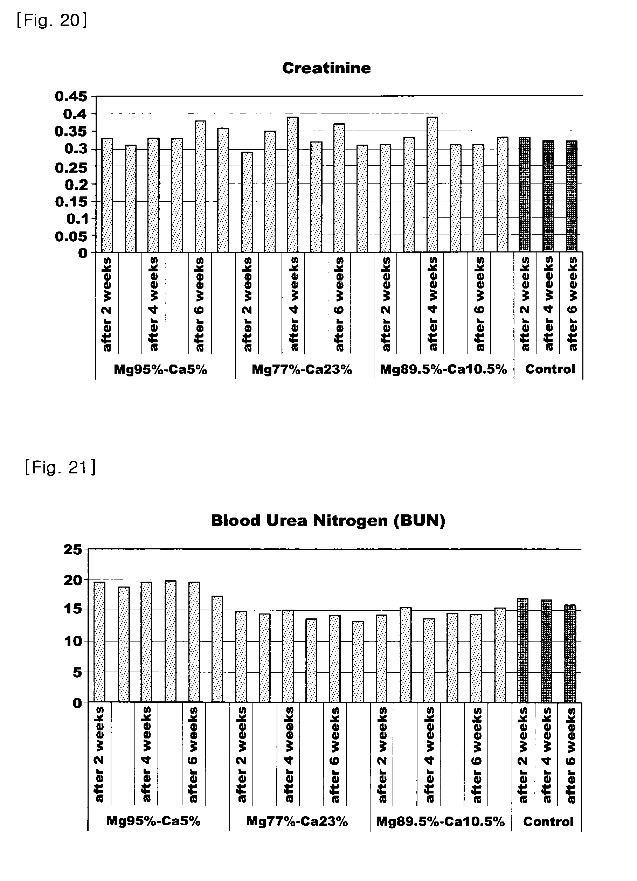

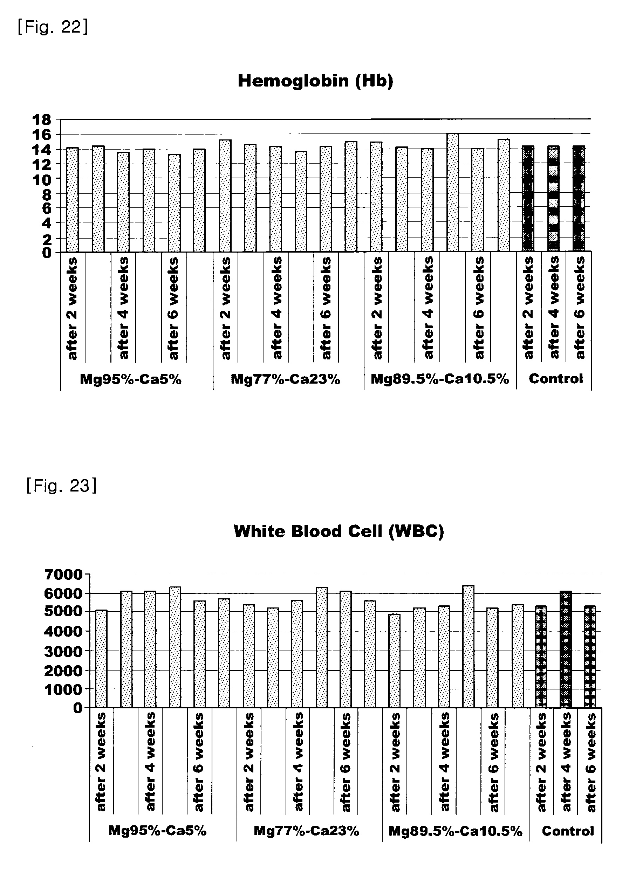

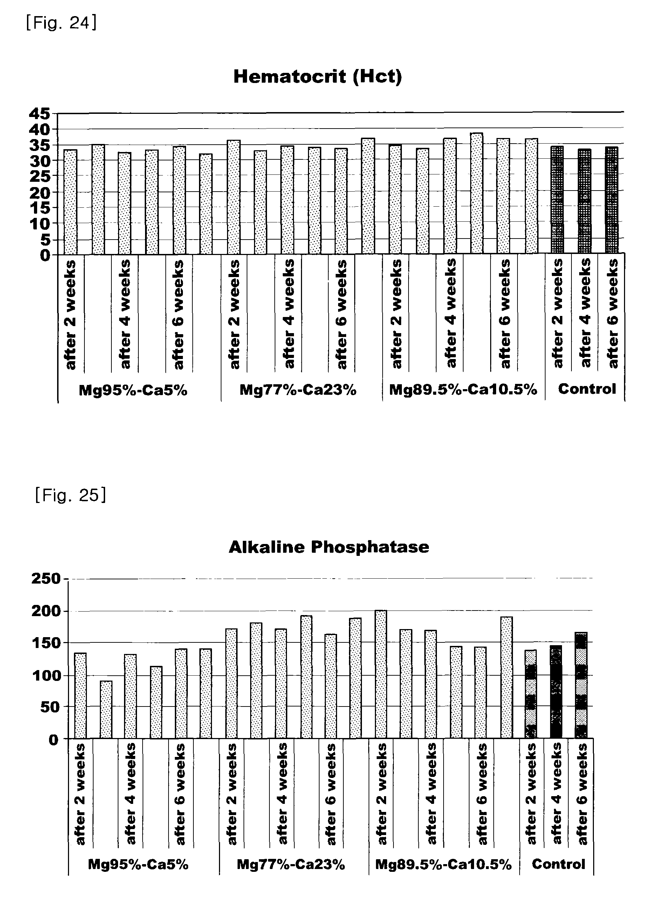

The magnesium-based alloy manufactured in Example 2 was applied to in vivo test using a mouse, and after 2 weeks, the bone formation was found in the region, in which the magnesium-based alloy was degraded (Fig. 17). Further, the magnesium-based alloy manufactured in Example 2 was applied to in vivo test using a mouse, and then blood was taken from the mouse to measure the changes in the levels of aspartate liver enzyme (AST), alanine liver enzyme (ALT), creatinine, blood urea nitrogen (BUN), hemoglobin, white blood cell, hematocrit (Hct), and alkaline phosphatase after 2 weeks, 4 weeks, and 6 weeks. The relative levels measured were shown in Figs. 18 to 25. From Figs. 18 to 25, it was found that there is no difference in systemic reactions, when a group grafted with the magnesium-based alloy was relatively compared with a control not grafted with the magnesium-based alloy.

## Claims

1. An implant comprising a biodegradable magnesium-based alloy, wherein the biodegradable magnesium-based alloy is represented by Formula MgₐCa_{b}X_{c}, wherein a, b and c are a molar ratio of each component, satisfying the following conditions: 0.5 ≤ a ≤ 1, 0 < b ≤ 0.4 and 0 < c ≤ 0.4, and X being nickel (Ni) and iron (Fe) which are comprised in an amount of up to 0.01 % Ni and 0.1 % Fe.

2. The implant according to claim 1, wherein the implant is an orthopedic, dental, plastic surgical or vascular implant.

3. The implant according to claim 1, wherein the implant is totally composed of a biodegradable magnesium-based alloy.

4. The implant according to claim 1, wherein the implant is provided with a coating layer composed of a biodegradable magnesium-based alloy on the surface.

5. A method for manufacturing an implant using a biodegradable magnesium-based alloy, wherein the biodegradable magnesium-based alloy is represented by Formula MgₐCa_{b}X_{c}, wherein a, b and c are a molar ratio of each component, satisfying the following conditions: 0.5 ≤ a ≤ 1, 0 < b ≤ 0.4 and 0 < c ≤ 0.4, and X being nickel (Ni) and iron (Fe) which are added in an amount of up to 0.01 % Ni and 0.1 % Fe,
comprising the steps of a) melting the biodegradable magnesium-based alloy, and b) molding the molten biodegradable magnesium-based alloy.

6. The method according to claim 5, wherein the step b) is a step of using an extrusion process.

7. The method according to claim 5, comprising the step of coating the base material surface of the implant with the magnesium-based alloy.

## Patentansprüche

1. Ein Implantat enthaltend eine biologisch abbaubare, Magnesium-basierte Legierung, wobei die biologisch abbaubare, Magnesium-basierte Legierung durch die Formel MgₐCa_{b}X_{c} dargestellt wird, wobei a, b und c ein molares Verhältnis jeder Komponente sind, das die folgenden Bedingungen erfüllt: 0,5 ≤ a ≤ 1, 0 < b ≤ 0,4 und 0 < c ≤ 0,4 und X Nickel (Ni) und Eisen (Fe) ist, welche in einer Menge von bis zu 0,01% Ni und 0,1% Fe enthalten sind.

2. Das Implantat gemäß Anspruch 1, wobei das Implantat ein orthopädisches, dentales, plastisch-chirurgisches oder vaskuläres Implantat ist.

3. Das Implantat gemäß Anspruch 1, wobei das Implantat vollständig aus einer biologisch abbaubaren, Magnesium-basierten Legierung besteht.

4. Das Implantat gemäß Anspruch 1, wobei das Implantat auf der Oberfläche mit einer Deckschicht aus einer biologisch abbaubaren, Magnesium-basierten Legierung ausgestattet ist.

5. Ein Verfahren zur Herstellung eines Implantats unter Verwendung einer biologisch abbaubaren, Magnesium-basierten Legierung, wobei die biologisch abbaubare, Magnesium-basierte Legierung durch die Formel MgₐCa_{b}X_{c} dargestellt wird, wobei a, b und c ein molares Verhältnis jeder Komponente sind, das die folgenden Bedingungen erfüllt: 0,5 ≤ a ≤ 1, 0 < b ≤ 0,4 und 0 < c ≤ 0,4 und X Nickel (Ni) und Eisen (Fe) ist, welche in einer Menge von bis zu 0,01% Ni und 0,1% Fe zugegeben werden,
enthaltend die Schritte a) Schmelzen der biologisch abbaubaren, Magnesium-basierten Legierung und b) Formen der geschmolzenen biologisch abbaubaren, Magnesiumbasierten Legierung.

6. Das Verfahren gemäß Anspruch 5, wobei der Schritt b) ein Schritt der Verwendung eines Extrusionsprozesses ist.

7. Das Verfahren gemäß Anspruch 5, enthaltend den Schritt des Beschichtens der Oberfläche des Basismaterials des Implantats mit der Magnesium-basierten Legierung.

## Revendications

1. Implant comprenant un alliage biodégradable à base de magnésium, dans lequel ledit alliage biodégradable à base de magnésium est représenté par la formule Mgₐ₋Ca_{b}X_{c}, dans laquelle a, b et c sont un rapport molaire de chaque composant, satisfaisant les conditions suivantes: 0,5 ≤ a ≤ 1,0 < b ≤ 0,4 et 0 < c ≤ 0,4, et X est du nickel (Ni) et du fer (Fe) qui sont contenus en une quantité allant jusqu'à 0,01 % de Ni et 0,1 % de Fe.

2. Implant selon la revendication 1, dans lequel l'implant est un implant orthopédique, dentaire, plastique chirurgical ou vasculaire.

3. Implant selon la revendication 1, dans lequel l'implant est entièrement composé d'un alliage biodégradable à base de magnésium.

4. Implant selon la revendication 1, dans lequel l'implant est pourvu, sur la surface, d'une couche de revêtement composée d'un alliage biodégradable à base de magnésium.

5. Procédé de fabrication d'un implant en utilisant un alliage biodégradable à base de magnésium, dans lequel ledit alliage biodégradable à base de magnésium est représenté par la formule MgₐCa_{b}X_{c}, dans laquelle a, b et c sont un rapport molaire de chaque composant, satisfaisant les conditions suivantes: 0,5 ≤ a ≤ 1,0 < b ≤ 0,4 et 0 < c ≤ 0,4, et X est du nickel (Ni) et du fer (Fe) qui sont ajoutés en une quantité allant jusqu'à 0,01 % de Ni et 0,1 % de Fe,
comprenant les étapes consistant a) à fondre ledit alliage biodégradable à base de magnésium et b) à mouler l'alliage fondu biodégradable à base de magnésium.

6. Procédé selon la revendication 5, dans lequel l'étape b) est une étape d'utilisation d'un processus d'extrusion.

7. Procédé selon la revendication 5, comprenant l'étape consistant à revêtir, dudit alliage à base de magnésium, la surface du matériau de base de l'implant.
